# FASCICULE DE BREVET EUROPEEN

(11) **EP 0 795 320 B1**
(45) Date de publication et mention de la délivrance du brevet: **20.01.1999**
(21) Numéro de dépôt: 97400178.6
(22) Date de dépôt: 27.01.1997
(51) Int. Cl.: A61K 7/48, C08L 5/14, A61K 47/36, A61K 7/06

(54) **Composition gélifiée stable à forte teneur en électrolyte**
Stabile gelierte Zusammensetzung mit hohem Elektrolytgehalt
Stable gelified composition with high electrolyte content

(30) Priorité: 12.03.1996 FR 9603094
(43) Date de publication de la demande: 17.09.1997
(73) Titulaire: L'OREAL, 75008 Paris (FR)
(72) Inventeur: Gagnebien, Didier, Westfield 07090, New-Jersey (US)
(74) Mandataire: Lhoste, Catherine

(56) Documents cités:
- EP-A- 0 281 360
- EP-A- 0 682 936
- EP-A- 0 708 114
- RESEARCH DISCLOSURE, no. 37807, Octobre 1995, page 642 XP002018143 MAJEWICZ ET AL.: "oil-based cosmetic and therapeutic compositions containing ethylguar"
- RESEARCH DISCLOSURE, no. 38413, 10 Avril 1996, pages 235-236, XP002033267 MAJEWICZ ET AL.: "ethyl galactomannan film properties for use in personal care applications"

## Description

La présente invention se rapporte à une composition gélifiée stable, notamment topique, pouvant contenir une quantité importante d'électrolyte, et à son utilisation notamment pour le traitement et le soin de la peau, du cuir chevelu, des muqueuses, des ongles et des cheveux.

Dans les domaines cosmétique, dermatologique et pharmaceutique, il est connu d'utiliser des compositions topiques sous forme de gels ou d'émulsions comportant des gélifiants qui donnent de la consistance à ces compositions. La majorité des gélifiants utilisés classiquement sont des gélifiants aqueux et notamment des polymères carboxyvinyliques, que l'on neutralise par une base.

Il arrive cependant que certains composés que l'on souhaite utiliser dans ces compositions ne permettent pas l'emploi des gélifiants cités précédemment par suite d'incompatibilité.

On sait par exemple que les électrolytes (sels inorganiques et organiques) "cassent" les émulsions gélifiées avec les polymères carboxyvinyliques et les liquéfient. Ainsi, des compositions contenant des polymères carboxyvinyliques et des électrolytes sont dépourvues de consistance, ce qui va à l'encontre du résultat recherché par l'emploi d'un gélifiant.

Or, il peut être souhaitable d'introduire des électrolytes dans des compositions épaissies, notamment topiques, et parfois même en une quantité relativement importante, notamment quand ces électrolytes ont un effet bénéfique sur la peau ou les cheveux.

Une solution consiste à utiliser, à la place des polymères carboxyvinyliques, des gélifiants de type polysaccharidique tels que les gommes guar ou xanthane ou les dérivés cellulosiques. Ainsi, le document EP-A-654270 décrit une composition topique destinée au traitement de l'acné et des dermatites séborrhéiques, comportant un mélange de sels et, comme gélifiant, un dérivé de cellulose tel que l'hydroxyéthylcellulose.

Malheureusement, les compositions à base de dérivés de cellulose conformes à ce document, et notamment les gels aqueux ne comportant pas de phase grasse, n'ont pas une texture lisse et présentent au contraire un aspect grumeleux peu agréable à voir. En outre, ils laissent la peau « comme mouillée » après application, ces compositions ne pénétrant pas suffisamment dans la peau. Tout ceci rend leur utilisation rédhibitoire dans les domaines cosmétique et/ou dermatologique.

L'association de ces dérivés de cellulose avec un autre agent épaississant tel qu'un silicate, comme décrit par exemple dans le document WO-A-93/8230, donne des compositions où subsistent les mêmes inconvénients qu'indiqués ci-dessus (aspect grumeleux).

II subsiste donc le besoin de compositions gélifiées contenant des électrolytes et ne présentant pas les inconvénients rencontrés avec les gélifiants connus, notamment d'inconsistance, d'instabilité, d'aspect grumeleux, de sensation désagréable au toucher et d'incompatibilité avec ces électrolytes.

La demanderesse a trouvé de façon inattendue une classe de gélifiants permettant de stabiliser les compositions contenant une forte teneur en électrolyte et en huile.

Aussi, la présente invention a pour objet une composition comportant au moins un électrolyte et une phase huileuse contenant au moins un gélifiant, le dit gélifiant étant un alkyléther de polysaccharide formé de motifs comportant au moins deux cycles osidiques différents, chaque motif comportant au moins un groupe hydroxyle substitué par une chaîne alkyle hydrocarbonée saturée.

Le gélifiant selon l'invention est un gélifiant d'huile, c'est-à-dire apte à épaissir les phases huileuses.

La composition selon l'invention peut être aussi bien un gel huileux qu'une émulsion eau-dans-huile ou huile-dans-eau ou une dispersion d'huile dans l'eau avec des vésicules lipidiques.

Selon un mode de réalisation particulier de l'invention, l'alkyléther de polysaccharide a un poids moléculaire supérieur à 100 000, et de préférence supérieur à 200 000. Chaque motif peut comporter de un à six et de préférence de deux à quatre groupes hydroxyle substitués par une chaîne alkyle hydrocarbonée saturée.

Par chaîne alkyle hydrocarbonée saturée, on entend une chaîne comportant de 1 à 24, de préférence de 1 à 10 et mieux de 1 à 5 atomes de carbone. En particulier, la chaîne alkyle est choisie parmi les chaînes méthyle, éthyle, n-propyle, isopropyle, n-butyle, isobutyle, tertio-butyle, n-pentyle.

Les cycles osidiques sont notamment choisis parmi le mannose, le galactose, le glucose, le furanose, le rhamnose, l'arabinose.

Selon un mode préféré de réalisation de l'invention, l'alkyléther de polysaccharide selon l'invention est un alkyléther d'une gomme et plus particulièrement d'une gomme globalement non ionique, c'est-à-dire pratiquement sans groupe ionique. Comme gommes appropriées, on peut citer par exemple la gomme guar dont le motif comprend un galactose et un mannose, la gomme de caroube dont le motif comprend un galactose et un mannose, la gomme de karaya qui est un mélange complexe de rhamnose, galactose et acide galacturonique, la gomme adragante qui est un mélange complexe d'arabinose, galactose et acide galacturonique.

Selon un mode de réalisation préféré de l'invention, l'alkyléther de polysaccharide est un dérivé de gomme guar, et plus particulièrement la guar éthylée ayant un degré de substitution d'environ 2 à 3, en particulier 2,5, telle que décrite dans le document RD 95378007 (octobre 1995).

La composition selon l'invention peut contenir par exemple une quantité d'alkyléther de polysaccharide allant de 0,5 à 10 %, et de préférence de 2 à 8 % du poids total de la composition.

L'électrolyte peut être présent dans la composition notamment en une quantité allant de 0,5 à 40 % en poids, et de préférence de 1 à 20 % en poids par rapport au poids total de la composition.

Comme électrolyte utilisable dans la composition selon l'invention, on peut citer notamment les sels des métaux mono-, di- ou trivalents, et plus particulièrement les sels de métal alcalino-terreux et en particulier les sels de baryum, de calcium et de strontium, les sels de métal alcalin et par exemple les sels de sodium et de potassium, ainsi que les sels de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

Les ions constituant ces sels peuvent être choisis par exemple parmi les carbonates, les bicarbonates, les sulfates, les glycérophosphates, les borates, les chlorures, les nitrates, les acétates, les hydroxydes, les persulfates ainsi que les sels d'α-hydroxyacides (citrates, tartrates, lactates, malates) ou d'acides de fruits, ou encore les sels d'acides aminés (aspartate, arginate, glycocholate, fumarate).

De préférence, le sel est choisi parmi le nitrate de calcium, de magnésium ou de strontium, le borate de calcium ou de magnésium, le chlorure de calcium, de sodium, de magnésium, de strontium, de néodyme ou de manganèse, le sulfate de magnésium ou de calcium, l'acétate de calcium ou de magnésium, et leurs mélanges.

La composition selon l'invention s'applique dans tous les domaines où l'on souhaite obtenir une composition épaissie en présence d'électrolytes et notamment dans le domaine des peintures, dans les domaines agro-alimentaire, cosmétique, dermatologique et pharmaceutique.

De préférence, la composition selon l'invention est destinée à un soin ou un traitement topique. Dans ce cas, la composition doit contenir un milieu topiquement acceptable, c'est-à-dire compatible avec la peau, les muqueuses, les ongles, le cuir chevelu et les cheveux. Elle peut se présenter sous toutes les formes galéniques appropriées pour une application topique, et notamment sous forme d'émulsion eau-dans-huile ou huile-dans-eau ou de dispersion huileuse contenant une faible quantité d'eau chargée en électrolytes, dispersée dans la phase huileuse. La composition selon l'invention peut aussi contenir des vésicules lipidiques ioniques et/ou non-ioniques, contenant ou non une huile dispersée. Elle peut constituer par exemple une crème ou une pommade.

Les quantités des différents constituants des compositions selon l'invention sont celles classiquement utilisées dans le domaine considéré.

Lorsque la composition est une dispersion huileuse, la proportion d'eau dispersée dans l'huile est inférieure à 10 % et de préférence inférieure à 5 %. L'eau dispersée peut contenir une quantité d'électrolytes allant jusqu'à la saturation.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

De façon connue, la composition de l'invention peut contenir également des adjuvants habituels dans les domaines cosmétique et/ou dermatologique, tels que les actifs, les conservateurs, les antioxydants, les agents complexants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme huiles utilisables dans l'invention, on peut citer par exemple les huiles d'origine végétale, les huiles d'origine animale, les huiles de synthèse et notamment les esters gras, et les mélanges de ces huiles, ainsi que les mélanges de ces huiles avec les huiles siliconées, les huiles fluorées et/ou les huiles minérales. On peut aussi utiliser comme matières grasses des alcools gras (alcool cétylique), des acides gras, des cires et composés cireux.

Comme émulsionnants utilisables dans l'invention, on peut citer par exemple le mélange de monostéarate de glycéryle et de stéarate de polyéthylèneglycol vendu par la société ICI sous la dénomination Arlacel 165.

Comme actifs utilisables dans l'invention, on peut citer par exemple les hydratants tels que la glycérine, les actifs pour traiter les signes du vieillissement, l'acné, les dermatites, les taches pigmentaires.

La composition selon l'invention peut notamment être utilisée pour le traitement et le soin de la peau, des muqueuses, du cuir chevelu, des ongles et/ou des cheveux, en particulier pour le traitement de la peau sensible et du cuir chevelu sensible et/ou pour hydrater la peau.

Aussi, la présente invention a encore pour objet l'utilisation de la composition telle que définie ci-dessus dans ou pour la fabrication d'une composition cosmétique, pharmaceutique et/ou dermatologique pour traiter la peau sensible et/ou le cuir chevelu sensible et/ou pour hydrater la peau. Pour de plus amples détails concernant la peau sensible, on peut se référer au document EP-A-680749.

La présente invention a aussi pour objet un procédé de traitement cosmétique et/ou dermatologique de la peau, du cuir chevelu, des cheveux, des ongles et/ou des muqueuses, caractérisé par le fait que l'on applique sur la peau, le cuir chevelu, les cheveux, les ongles et/ou les muqueuses, une composition telle que définie ci-dessus.

L'exemple ci-après de composition selon l'invention, est un exemple hypothétique réalisable donné à titre d'illustration et sans caractère limitatif. Les quantités y sont données en % en poids.

### Exemple : Emulsion huile-dans-eau

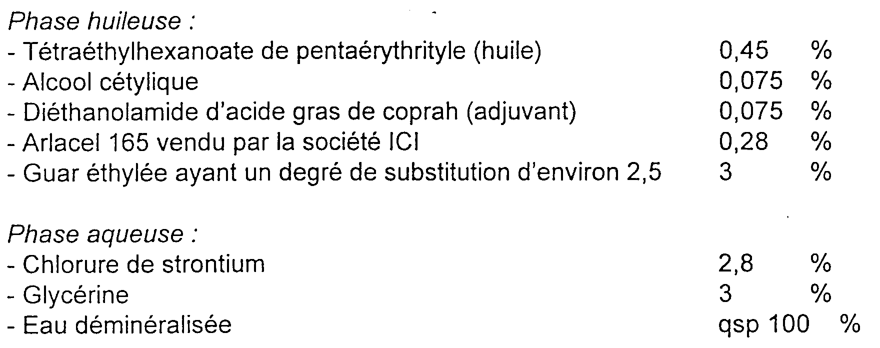

La crème obtenue pourrait être notamment utilisée pour le traitement des peaux sensibles.

## Revendications

1. Composition comportant au moins un électrolyte et une phase huileuse contenant au moins un gélifiant, le dit gélifiant étant un alkyléther de polysaccharide formé de motifs comportant au moins deux cycles osidiques différents, chaque motif comportant au moins un groupe hydroxyle substitué par une chaîne alkyle hydrocarbonée saturée.

2. Composition selon la revendication 1, caractérisée en ce que chaque motif comporte deux à quatre groupes hydroxyle substitués par une chaîne alkyle hydrocarbonée saturée.

3. Composition selon la revendication 1 ou 2, caractérisée en ce que la chaîne alkyle hydrocarbonée saturée comporte de 1 à 24 atomes de carbone.

4. Composition selon l'une quelconque des revendications précédentes, caractérisée en ce que la chaîne alkyle hydrocarbonée saturée comporte de 1 à 5 atomes de carbone.

5. Emulsion selon l'une des revendications précédentes, caractérisée en ce que la chaîne alkyle est choisie parmi les chaînes méthyle, éthyle, n-propyle, isopropyle, n-butyle, isobutyle, tertiobutyle, n-pentyle.

6. Composition selon l'une quelconque des revendications précédentes, caractérisée en ce que les cycles osidiques sont choisis parmi le mannose, le galactose, le glucose, le furanose, le rhamnose, l'arabinose.

7. Composition selon l'une quelconque des revendications précédentes, caractérisée en ce que l'alkyléther de polysaccharide est un alkyléther d'une gomme choisie parmi la gomme guar, la gomme de caroube, la gomme de karaya, la gomme adragante et leurs mélanges.

8. Composition selon l'une quelconque des revendications précédentes, caractérisée en ce que l'alkyléther de polysaccharide a un poids moléculaire supérieur à 200 000.

9. Composition selon l'une quelconque des revendications précédentes, caractérisée en ce que l'alkyléther de polysaccharide est présent en une quantité allant de 0,5 à 10 % du poids total de la composition.

10. Composition selon l'une quelconque des revendications précédentes, caractérisée en ce que l'alkyléther de polysaccharide est présent en une quantité allant de 2 à 8 % du poids total de la composition.

11. Composition selon l'une quelconque des revendications précédentes, caractérisée en ce que l'électrolyte est présent en une concentration allant de 0,5 à 40 % en poids par rapport au poids total de la composition.

12. Composition selon l'une quelconque des revendications précédentes, caractérisée en ce que l'électrolyte est un sel de métal mono-, di-ou trivalent.

13. Composition selon la revendication précédente, caractérisée en ce que le sel est choisi parmi les sels de baryum, de calcium, de strontium, de sodium, de potassium, de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

14. Composition selon l'une quelconque des revendications précédentes, caractérisée en ce que l'électrolyte est constitué d'ions choisis parmi les ions chlorure, borate, bicarbonate, carbonate, nitrate, hydroxyde, sulfate, persulfate, glycérophosphate, acétate, d'α-hydroxyacides, d'acides de fruit, d'acides aminés.

15. Composition selon l'une quelconque des revendications précédentes, caractérisée en ce que l'électrolyte est choisi parmi le nitrate de calcium, de magnésium ou de strontium, le borate de calcium ou de magnésium, le chlorure de calcium, de magnésium, de sodium, de strontium, de néodyme ou de manganèse, le sulfate de magnésium ou de calcium, l'acétate de calcium ou de magnésium, et leurs mélanges.

16. Composition selon l'une quelconque des revendications précédentes, caractérisée en ce qu'elle est une dispersion huileuse, une émulsion eau-dans-huile ou une émulsion huile-dans-eau.

17. Composition selon l'une quelconque des revendications précédentes, caractérisée en ce qu'elle constitue une composition topique.

18. Composition selon la revendication précédente, caractérisée en ce qu'elle contient un actif cosmétique et/ou dermatologique.

19. Utilisation de la composition selon l'une quelconque des revendications précédentes pour la fabrication d'une composition cosmétique, pharmaceutique et/ou dermatologique pour traiter la peau sensible et/ou le cuir chevelu sensible et/ou pour hydrater la peau.

20. Procédé de traitement cosmétique de la peau, du cuir chevelu, des cheveux, des ongles et/ou des muqueuses, caractérisé par le fait que l'on applique sur la peau, le cuir chevelu, les cheveux, les ongles et/ou les muqueuses, une composition selon l'une quelconque des revendications 1 à 18.

## Claims

1. Composition containing at least one electrolyte and an oily phase containing at least one gelling agent, the said gelling agent being a polysaccharide alkyl ether formed from units containing at least two different monosaccharide rings, each unit containing at least one hydroxyl group substituted by a saturated hydrocarbon alkyl chain.

2. Composition according to Claim 1, characterized in that each unit contains two to four hydroxyl groups substituted by a saturated hydrocarbon alkyl chain.

3. Composition according to Claim 1 or 2, characterized in that the saturated hydrocarbon alkyl chain contains from 1 to 24 carbon atoms.

4. Composition according to any one of the preceding claims, characterized in that the saturated hydrocarbon alkyl chain contains from 1 to 5 carbon atoms.

5. Emulsion according to one of the preceding claims, characterized in that the alkyl chain is chosen from methyl, ethyl, n-propyl, isopropyl, n-butyl, isobutyl, tert-butyl and n-pentyl chains.

6. Composition according to any one of the preceding claims, characterized in that the monosaccharide rings are chosen from mannose, galactose, glucose, furanose, rhamnose and arabinose.

7. Composition according to any one of the preceding claims, characterized in that the polysaccharide alkyl ether is an alkyl ether of a gum chosen from guar gum, locust bean gum, karaya gum, gum tragacanth and their mixtures.

8. Composition according to any one of the preceding claims, characterized in that the polysaccharide alkyl ether has a molecular weight of greater than 200,000.

9. Composition according to any one of the preceding claims, characterized in that the polysaccharide alkyl ether is present in an amount ranging from 0.5 to 10% of the total weight of the composition.

10. Composition according to any one of the preceding claims, characterized in that the polysaccharide alkyl ether is present in an amount ranging from 2 to 8% of the total weight of the composition.

11. Composition according to any one of the preceding claims, characterized in that the electrolyte is present in a concentration ranging from 0.5 to 40% by weight with respect to the total weight of the composition.

12. Composition according to any one of the preceding claims, characterized in that the electrolyte is a salt of a mono-, di- or trivalent metal.

13. Composition according to the preceding claim, characterized in that the salt is chosen from barium, calcium, strontium, sodium, potassium, magnesium, beryllium, yttrium, lanthanum, cerium, praseodymium, neodymium, promethium, samarium, europium, gadolinium, terbium, dysprosium, holmium, erbium, thulium, ytterbium, lutetium, lithium, tin, zinc, manganese, cobalt, nickel, iron, copper, rubidium, aluminium, silicon and selenium salts, and their mixtures.

14. Composition according to any one of the preceding claims, characterized in that the electrolyte is composed of ions chosen from chloride, borate, bicarbonate, carbonate, nitrate, hydroxide, sulphate, persulphate, glycerophosphate and acetate ions or ions of α-hydroxy acids, of fruit acids or of amino acids.

15. Composition according to any one of the preceding claims, characterized in that the electrolyte is chosen from calcium, magnesium or strontium nitrate, calcium or magnesium borate, calcium, magnesium, sodium, strontium, neodymium or manganese chloride, magnesium or calcium sulphate, calcium or magnesium acetate, and their mixtures.

16. Composition according to any one of the preceding claims, characterized in that it is an oily dispersion, a water-in-oil emulsion or an oil-in-water emulsion.

17. Composition according to any one of the preceding claims, characterized in that it constitutes a topical composition.

18. Composition according to the preceding claim, characterized in that it contains a cosmetic and/or dermatological active principle.

19. Use of the composition according to any one of the preceding claims for the manufacture of a cosmetic, pharmaceutical and/or dermatological composition for treating sensitive skin and/or sensitive scalps and/or for moisturizing the skin.

20. Process for the cosmetic treatment of the skin, scalp, hair, nails and/or mucous membranes, characterized in that a composition according to any one of Claims 1 to 18 is applied to the skin, scalp, hair, nails and/or mucous membranes.

## Patentansprüche

1. Zusammensetzungen, die mindestens einen Elektrolyt und eine Ölphase, welche mindestens einen Gelbildner enthält, aufweisen, wobei dieser Gelbildner ein Polysaccharidalkylether ist, der aus Einheiten besteht, welche mindestens zwei verschiedene Zuckerringe aufweisen, wobei jede Einheit mindestens eine Hydroxygruppe aufweist, die mit einer Alkylgruppe (gesättigte Kohlenwasserstoffgruppe) substituiert ist.

2. Zusammensetzungen nach Anspruch 1, dadurch gekennzeichnet, daß jede Einheit zwei bis vier Hydroxygruppen aufweist, die mit einer Alkylgruppe substituiert sind.

3. Zusammensetzungen nach Anspruch 1 oder 2, dadurch gekennzeichnet, daß die Alkylgruppe 1 bis 24 Kohlenstoffatome aufweist.

4. Zusammensetzungen nach einem der vorhergehenden Ansprüche, dadurch gekennzeichnet, daß die Alkylgruppe 1 bis 5 Kohlenstoffatome aufweist.

5. Emulsionen nach einem der vorhergehenden Ansprüche, dadurch gekennzeichnet, daß die Alkylgruppe unter Methyl, Ethyl, n-Propyl, Isopropyl, n-Butyl, Isobutyl, *tert*.-Butyl und n-Pentyl ausgewählt ist.

6. Zusammensetzungen nach einem der vorhergehenden Ansprüche, dadurch gekennzeichnet, daß die Zuckerringe unter Mannose, Galactose, Glucose, Furanose, Rhamnose und Arabinose ausgewählt sind.

7. Zusammensetzungen nach einem der vorhergehenden Ansprüche, dadurch gekennzeichnet, daß der Polysaccharidalkylether ein Alkylether eines Gummis ist, welches unter Guargummi, Johannisbrotkernmehl, Karaya-Gummi, Tragant und den Gemischen dieser Gummen ausgewählt ist.

8. Zusammensetzungen nach einem der vorhergehenden Ansprüche, dadurch gekennzeichnet, daß der Polysaccharidalkylether ein Molekulargewicht von über 200 000 aufweist.

9. Zusammensetzungen nach einem der vorhergehenden Ansprüche, dadurch gekennzeichnet, daß der Polysaccharidalkylether in einem Mengenanteil im Bereich von 0,5 bis 10 % des Gesamtgewichts der Zusammensetzung vorliegt.

10. Zusammensetzungen nach einem der vorhergehenden Ansprüche, dadurch gekennzeichnet, daß der Polysaccharidalkylether in einem Mengenanteil im Bereich von 2 bis 8 % des Gesamtgewichts der Zusammensetzung vorliegt.

11. Zusammensetzungen nach einem der vorhergehenden Ansprüche, dadurch gekennzeichnet, daß der Elektrolyt in einer Konzentration im Bereich von 0,5 bis 40 Gew.-%, bezogen auf das Gesamtgewicht der Zusammensetzung, vorliegt.

12. Zusammensetzungen nach einem der vorhergehenden Ansprüche, dadurch gekennzeichnet, daß es sich bei dem Elektrolyt um ein einwertiges, zweiwertiges oder dreiwertiges Metallsalz handelt.

13. Zusammensetzungen nach dem vorhergehenden Anspruch, dadurch gekennzeichnet, daß das Salz ausgewählt ist unter den Salzen von Barium, Calcium, Strontium, Natrium, Kalium, Magnesium, Beryllium, Yttrium, Lanthan, Cer, Praseodym, Neodym, Promethium, Samarium, Europium, Gadolinium, Terbium, Dysprosium, Holmium, Erbium, Thulium, Ytterbium, Lutetium, Lithium, Zinn, Zink, Mangan, Cobalt, Nickel, Eisen, Kupfer, Rubidium, Aluminium, Silicium, Selen und deren Gemischen.

14. Zusammensetzungen nach einem der vorhergehenden Ansprüche, dadurch gekennzeichnet, daß der Elektrolyt aus Ionen gebildet ist, die ausgewählt sind unter Chlorid, Borat, Bicarbonat, Carbonat, Nitrat, Hydroxid, Sulfat, Persulfat, Glycerophosphat, Acetat, Ionen von α-Hydroxysäuren, Ionen von Fruchtsäuren und Ionen von Aminosäuren.

15. Zusammensetzungen nach einem der vorhergehenden Ansprüche, dadurch gekennzeichnet, daR der Elektrolyt ausgewählt ist unter Calciumnitrat, Magnesiumnitrat oder Strontiumnitrat, Calciumborat oder Magnesiumborat, Calciumchlorid, Magnesiumchlorid, Natriumchlorid, Strontiumchlorid, Neodymchlorid oder Manganchlorid, Magnesiumsulfat oder Calciumsulfat, Calciumacetat oder Magnesiumacetat und deren Gemischen.

16. Zusammensetzungen nach einem der vorhergehenden Ansprüche, dadurch gekennzeichnet, daR es sich um ölige Dispersionen, Wasser-in-Öl-Emulsionen oder Öl-in-Wasser-Emulsionen handelt.

17. Zusammensetzungen nach einem der vorhergehenden Ansprüche, dadurch gekennzeichnet, daR sie topische Zusammensetzungen darstellen.

18. Zusammensetzungen nach dem vorhergehenden Anspruch, dadurch gekennzeichnet, daR sie einen kosmetischen und/oder dermatologischen Wirkstoff enthalten.

19. Verwendung der Zusammensetzungen nach einem der vorhergehenden Ansprüche zur Herstellung kosmetischer, pharmazeutischer und/oder dermatologischer Zusammensetzungen zur Behandlung empfindlicher Haut und/oder empfindlicher Kopfhaut und/oder zur Hydratisierung der Haut.

20. Verfahren zur kosmetischen Behandlung der Haut, der Kopfhaut, der Haare, der Nägel und/oder der Schleimhäute, dadurch gekennzeichnet, daR auf die Haut, die Kopfhaut, die Haare, die Nägel und/oder die Schleimhäute eine Zusammensetzung nach einem der Ansprüche 1 bis 18 aufgetragen wird.
